# EUROPEAN PATENT APPLICATION

(11) **EP 1 714 624 A1**
(43) Date of publication of application: **25.10.2006**
(21) Application number: 05007155.4
(22) Date of filing: 01.04.2005
(51) Int. Cl.: A61F 5/14, A43B 7/14

(54) **Foot orthosis**

(71) Applicant: Song, Ching-hui, Taipei 104 (TW)
(72) Inventor: Song, Ching-hui, Taipei 104 (TW); Tzeng, Txann-ywh, Taipei 104 (TW); Scott, Ma, Dr., Taipei 104 (TW)
(74) Representative: Jennings, Nigel Robin

(57) **Abstract**

A foot orthosis includes a shell (10) and corrective posts. The corrective posts (20,21,22,23,24,25) are attached to the shell through mechanical locking mechanism. The placement of post on the shell can be easily adjusted. Normal gait, optimal posture alignment, as well as prevention of musculoskeletal pain may be achieved through proper application of foot orthosis.

## Description

### 1. field of the Invention:

The present invention relates to foot orthosis, which may correct the faulty biomechanics of the lower extremities.

### 2. Description of Related Art

Ambulation depends on neuromusculoskeletal structures. If the skeletal structures are malaligned, walking can be a very painful experience. Most people have normal skeletal structures of their feet, so they are able to walk without significant problem, However, some people have abnormal skeletal structure associated with faulty biomechanics. For example, some individuals have lower limb length discrepancy, excessive foot pronation or supination. These abnormalities predispose to abnormal gait or musculoskeletal pain.

People wear shoes while walking to protect their feet from the external environment. However, most shoes do not contain any corrective features. For those who with abnormal feet, walking with regular shoes may be a problem because they cannot walk optimally without corrective shoes.

People with abnormal feet frequently suffer from musculoskeletal pain. Some podiatrists have invented exquisite foot orthosis to improve faulty biomechanics of lower limbs. Nevertheless, the foot orthosis has been custom made in a pedorthic lab, and the custom-made foot orthosis is time-consuming. To overcome the shortcomings of custom-made foot orthosis, the present invention provides a foot orthosis, which may partially resolve the aforementioned problems.

### Disclosure of invention

The primary objective of the present invention is to provide a foot orthosis, which may correct faulty biomechanics of foot. The purpose of the present invention is prefabricated orthosis. The cost of the foot orthosis can be reduced to a fraction of custom-made one so that most general consumers with abnormal feet can afford the foot orthosis. In addition, self adjustment may be achieved without professional assistance.

The foot orthosis consists of a shell and a corrective wedge assembly. The corrective wedge assembly is attached to the shell. Faulty biomechanics of foot may be improved via optimal application of the foot orthosis. Other objectives, advantages and novel features of the invention will become more apparent from the following detailed description along with the accompanying drawings.

### Brief Description of the Drawings

Fig. 1 is an exploded perspective view of a foot orthosis in accordance with the present invention.
Fig. 2 is a perspective view of the foot orthosis in Fig. 1.
Fig. 3 is a rear view in partial section of the second embodiment of a foot orthosis in accordance with the present invention.
Fig. 4 is a rear view in partial section of the third embodiment of the foot orthosis in accordance with the present invention.
Fig. 5 is a rear view in partial section of the fourth embodiment of the foot orthosis in accordance with the present invention.
Fig. 6 is a rear view in partial section of the fifth embodiment of the foot orthosis in accordance with the present invention.
Fig. 7 is a perspective view of the sixth embodiment of the foot orthosis in accordance with the present invention.
Fig. 8 is a perspective view of the seventh embodiment of the foot orthosis in accordance with the present invention.

### Detailed Description of the Preferred Embodiment

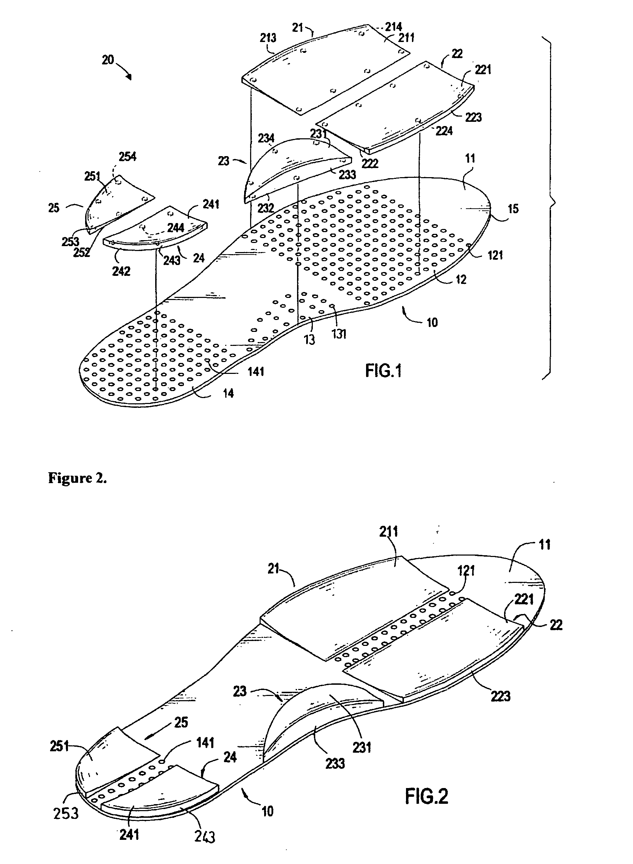

With reference to Figs. 1 and 2, the foot orthosis in accordance with the present invention consists of a shell (10) and a corrective wedge assembly (20).

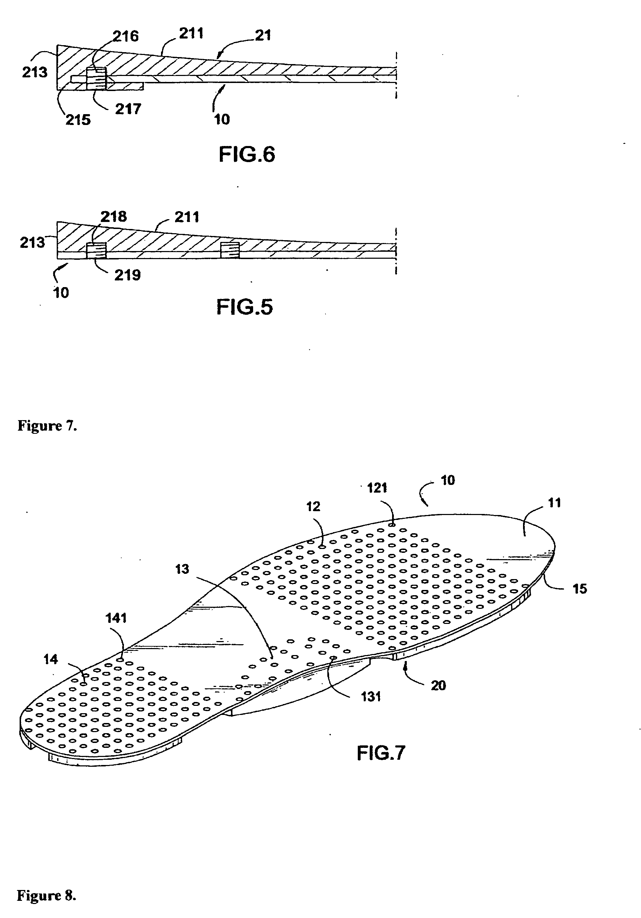

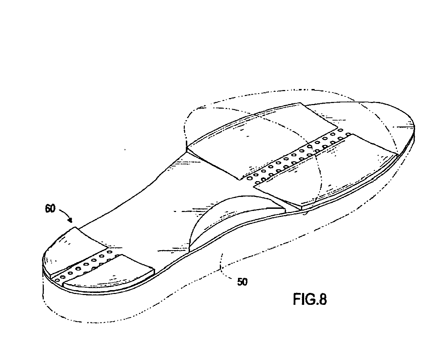

The shell (10) has a top surface (11), a bottom surface (15) and a base connection device. The top surface (11) has forefoot area (12), midfoot area (13), and hindfoot area (14). With further reference to Fig. 7, the base connection device is selectively on the top surface (11) or the bottom surface (15) of the shell (10).

With further reference to Figs. 1. 3, and 4, the base connection device may be receptive holes (121,131,141) or a hook connector (40);

With further reference to Fig. 1 and Fig. 6, the corrective wedge assembly (20) has top surface (211, 221, 231, 241, 251), bottom surface (212, 222, 232, 242, 252), curved sides (213, 223, 233, 243, 253), complementary connection devices, optional arms (215), and optional height adjusters (30), which are attached selectively to the shell (10) and include a lateral forefoot wedge (21), a medial forefoot wedge (22), an optional midfoot wedge (23), a medial hindfoot wedge (24) and a lateral hindfoot wedge (25).

The complementary connection devices are formed on the bottom surfaces (212, 222, 232, 242, 252) respectively of the corrective wedge assembly (20), which correspond and attach respectively to the base connection devices. Therefore, secure attachment of the corrective wedge assembly (20) to the shell (10) is achieved.

The arms (215) are attached to an edge of the curved sides (215, 223. 233, 243, 253). Receptive holes (not numbered) are formed on a bottom surface of the arms (215).

Since the base connection device comprises receptive holes (121,131,141), the complementary connection device comprises corresponding protrusions (214, 224, 234, 241, 254) that selectively engage the receptive holes (121,131,141) to securely attach the corrective wedge assembly (20) to the shell (10),

The complementary connection device is a hook connector (41) where the base connection device is a loop connector (40). The loop connectors (40) on the corrective wedge assembly (20) attach the corrective wedge assembly (20) to the shell (10) by engaging the hook connectors (41) on the shell (10).

With reference Fig. 1 and 5, the complementary connection device comprises screw receptive holes (218) in which the base connection device is receptive holes (121,131,141), the screw holes (218) and receptive holes (121, 131, 141) receive screws (219) that securely attach the corrective wedge assembly (20) to the shell (10).

With reference Fig. 6, the base connection device is receptive holes (121,131,141) where the complementary connection device comprises screw holes (218) and receptive holes in the arms (215). The screw holes (216), the receptive holes of the arms (215) and the receptive holes (121, 131. 141) receive screws (219) that securely attach the corrective wedge assembly (20) to the shell (10).

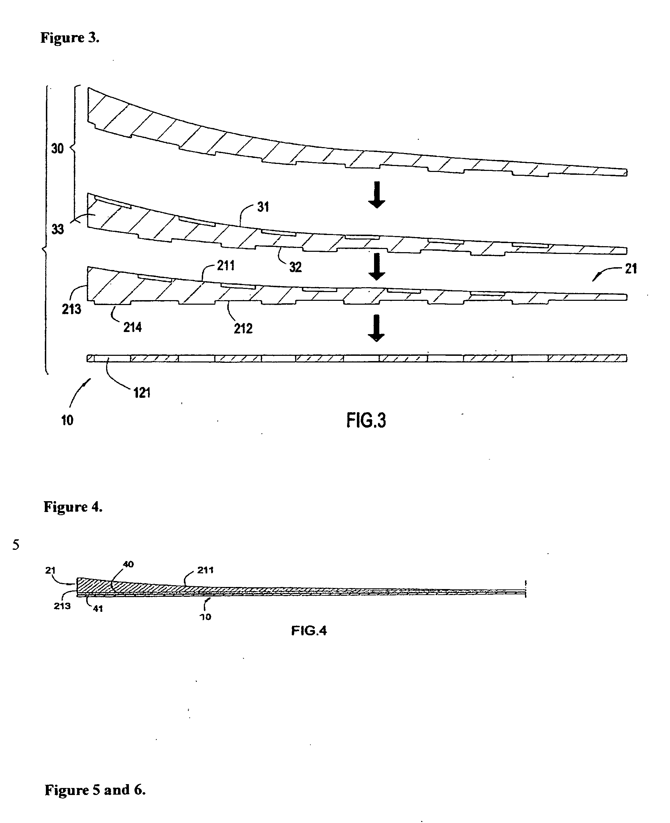

With reference Fig. 1 and 3, the height adjusters (30) have top surface (31), bottom surface (32), curved sides (33) and shapes corresponding to the corrective wedge assembly (20). The bottom surfaces (32) of the height adjusters (30) are selectively and respectively attached to the corresponding top surface (211, 221, 231, 241, 251) of the corrective wedge assembly (20), The height adjusters (30) can be stacked on each other to increase the height of the corrective wedge assembly (20). The lateral forefoot corrective wedge (21) and medial forefoot corrective wedge (22) are mounted on the forefoot area (12). The midfoot corrective wedge (23) is mounted on the midfoot area (18). The medial hindfoot corrector wedge (24) and the lateral hindfoot corrector wedge (25) are mounted on the hindfoot area (14).

People with abnormal feet can assemble the shell (10) with corrective wedge assembly (20) by themselves to fulfill their own specific needs. The corrective wedge assembly (20) may manipulate the joint motion to desired biomechanical function. Therefore, people with abnormal feet are able to walk smoothly and comfortably.

With reference Fig. 7, the corrective wedge assembly (20) can also able to be connected to the bottom-side surface (15) of the shell (10). The connection between the corrective wedge assembly (20) and the shell (10) has been displayed before.

With reference Fig. 8, in the sixth embodiment, the attachment that is displayed before is constructed as an insole (50). An insole is a layer of material shaped to the bottom of the bottom of the last and sandwiched between the outsole (.or mid-sole) and the sole of the foot inside the shoe). It is the shoe's structural anchor to which is attached the upper, box toe, lining, and welting. The corrective wedge assembly (60) is connected to the insole (50), which may provide people with abnormal feet comfort. The connection between the corrective wedge assembly (60) and the insole (50) are disclosed before.

Even though numerous characteristics and advantages of the present invention have been set forth in the foregoing description together with detailed illustration of the structure and function of the invention, the disclosure figures are for illustrative purpose only. Changes may be made in detail with regard to shape, size, and arrangement of parts within principles of the invention to the full extent indicated by the broad general meaning of the terms in which the appended claims are expressed.

## Claims

1. A foot orthosis comprise the following:
A shell having-
a top surface with a forefoot area, a midfoot area, and a hindfoot area. And a bottom surface, and a base connection device.
A corrective wedge assembly attached to the shell and having-
Top surfaces, bottom surfaces, curved sides, and a complementary connection device corresponding to and easily separate connected to the base connection device.
Wherein the corrective wedge assembly including :
A medial forefoot wedge connecting to the forefoot area of the shell.
A lateral forefoot wedge connecting to the forefoot area of the shell.
A medial hindfoot wedge connecting to the hindfoot area of the shell.
A lateral hindfoot wedge connecting to the hindfoot area of the shell.

2. The foot orthosis as claimed in claim 1, wherein the corrective wedge assembly has a midfoot wedge connecting to the midfoot area of the shell.

3. The foot orthosis as claimed in claim 2, wherein the base connection device is selectively provided on the top surface and the bottom surface of the shell.

4. The foot orthosis as claimed in claim 3, wherein the corrective wedge assembly has height adjuster having:
A top surface, a bottom surfaces selectively attached respectively to a corresponding of the top surfaces of the corrective wedge assembly.
A curve side.
A shape corresponding to the corrective wedge assembly.

5. The foot orthosis as claimed in claim 4, wherein the height adjusters is stacked on top of each other to increase height of the corrective wedge assembly.

6. The foot orthosis as claimed in claim 5, wherein the base connection device includes receptive holes in the shell and the complementary connection device includes protrusions that selectively extend into the receptive holes to securely attach the corrective wedge assembly to the shell.

7. The foot orthosis of claim 6, wherein the base connection device is a loop connector and the complementary connection device is a hook connector that attaches the corrective wedge assembly to the shell by the loop connector engaging the hook connector.

8. The foot orthosis of claim 7, wherein the base connection device is receptive holes, and the complementary connection device is screw holes. The receptive holes and screw holes receive screws that attach the corrective wedge assembly to the shell.

9. The foot orthosis of claim 8, wherein the corrective wedge assembly has arms that attach the edge of the curved sides, and the arms have receptive holes that are formed on bottom faces of the arms.

10. The foot orthosis of claim 9, wherein the base connection device is receptive holes when the complementary connection device comprises screw holes and receptive holes in the arms. The screw holes, the receptive holes of the arms, and the receptive holes receive screws that securely attach the corrective wedge assembly to the shell.
